# EUROPÄISCHE PATENTSCHRIFT

(11) **EP 3 041 435 B1**
(45) Veröffentlichungstag und Bekanntmachung des Hinweises auf die Patenterteilung: **29.05.2019**
(21) Anmeldenummer: 14761981.1
(22) Anmeldetag: 05.09.2014
(51) Int. Cl.: A61C 8/00, F16B 25/00, F16B 39/284

(54) **SCHRAUBE MIT ELLIPTISCHEM LÄNGS- UND QUERSCHNITT**
SCREW WITH AN ELLIPTICAL LONGITUDINAL AND CROSS SECTION
VIS À SECTION LONGITUDINALE ET TRANSVERSALE ELLIPTIQUE

(30) Priorität: 05.09.2013 DE 102013217734
(43) Veröffentlichungstag der Anmeldung: 13.07.2016
(73) Patentinhaber: CeramTec GmbH, 73207 Plochingen (DE)
(72) Erfinder: WECKER, Heinrich, 90542 Eckental (DE); KELNBERGER, Alfons, 90552 Röthenbach (DE); JUSZCZYK, Mateusz, 91235 Velden (DE)
(74) Vertreter: Fehrenbacher, Eckhard Anton
(86) Internationale Anmeldenummer: PCT/EP2014/068902
(87) Internationale Veröffentlichungsnummer: WO 2015/032883

(56) Entgegenhaltungen:
- WO-A1-95/08963
- DE-A1-102007 023 864
- GB-A- 1 218 836
- US-A- 2 414 870
- US-A- 3 763 909
- US-A- 6 039 568
- US-A1- 2004 254 575

## Beschreibung

Die Erfindung betrifft eine Knochenschraube. Insbesondere betrifft die Erfindung eine selbstsichernde Schraube.

Eine Schraube ist ein Stift oder Bolzen, der außen mit einem Gewinde versehen ist und einen Schraubenkopf mit einem Schraubenkopfantrieb aufweist. Eine mit einer Schraube hergestellte Verbindung ist formschlüssig und in der Regel wieder lösbar.

Es gibt eine Vielzahl verschiedener geometrischer Variationen bezüglich Gewindeformen und Schraubenkopf bzw. Schraubenkopfantriebe. Zur Sicherung von Schrauben, insbesondere zum Sichern gegen Lösen, sind ebenfalls eine Reihe von Techniken bekannt. Diese beruhen in der Regel darauf, dass der Schraubenkopf gegen nachträgliches Verdrehen gesichert wird. Beispiele für solche Schraubensicherungen sind Splinte, Gegenmuttern, verschiedenste Varianten von Ringscheiben und viele andere mehr.

Eine weitere Variante der Schraubensicherung besteht in der Verklemmung oder Verklebung des Gewindes der Schraube mit dem Werkstoff, in den die Schraube eingebracht ist. Dies kann durch einen zusätzlich eingebrachten Klebstoff oder durch eine Beschichtung des Schraubengewindes erreicht werden.

Diese Methoden der Schraubensicherung gegen eine nachträgliche Lockerung haben aber den Nachteil, dass sie Werkstoffe benötigen, die einen relativ hohen Elastizitätsmodul aufweisen, d.h. es werden Werkstoffe benötigt, die steif sind. Die Schrauben sollten dagegen einen geringeren Elastizitätsmodul aufweisen, also eher elastisch sein, um eine haltbare Verbindung zu erreichen. Ein anderer Nachteil besteht darin, dass die Schraube als solche nicht ausreicht, um eine Sicherung zu erreichen, sondern es werden üblicherweise andere Bauteile oder Materialien benötigt, um die Schraube zu sichern.

Außerdem können die Verbindungen aber bei Werkstoffen problematisch sein, die eher elastisch oder deformierbar reagieren. Hier kommt es schnell zu einem Ausschlagen der Schraublöcher und damit zu einer verminderten bzw. unzureichenden Reibung zwischen Schraube und Werkstoff. Dadurch kann sich eine Schraube leicht lockern.

Wenn jedoch eine zuverlässige Schraubverbindung absolut notwendig ist, kann eine solche Lockerung nicht toleriert werden. Ein Beispiel hierfür ist ein Implantat, das über eine Verschraubung fest mit einem Knochen verbunden werden soll. Ein Versagen der Schraubverbindung kann fatale Folgen haben, da in diesem Fall nicht nur eine weitere Operation notwendig wird, sondern auch sekundäre Verletzungen des umliegenden Gewebes durch das gelockerte Implantat entstehen können.

Aus GB1 218 836 sind selbstsichernde Schrauben mit im Längs- und Querrichtung elliptischen Schraubenkörper bekannt. In der US2004/254575 A1 werden Knochenschrauben gezeigt, die in Längsrichtung elliptisch ausgeformt sind.

Die Aufgabe der Erfindung besteht in der Bereitstellung einer Schraube, die ohne Zuhilfenahme anderer Bauteile eine dauerhaft sichere Verbindung mit einem Werkstoff, insbesondere mit einem elastischen Werkstoff, herstellt. Die Verbindung sollte vorzugsweise formschlüssig sein und sich nicht von selbst lösen. Vorzugsweise ist die hergestellte Verbindung aber wieder intentionell lösbar.

Diese Aufgabe wird durch eine Knochenschraube mit den Merkmalen gemäß Anspruch 1 gelöst. Die erfindungsgemäße Schraube weist einen Schraubenkörper mit einem Gewinde auf, wobei der Schraubenkörper elliptisch ist. Gemäß der Erfindung weist der Schraubenkörper einen elliptischen Querschnitt und einen elliptischen Längsschnitt auf.

Bei einem Schraubenkörper mit einem elliptischen Querschnitt hängt das Verhältnis der beiden elliptischen Halbachsen R/r und damit die Form des elliptischen Querschnitts im Wesentlichen von der Elastizität des Mediums ab, in das die Schraube geschraubt wird. "R" bezeichnet dabei die Länge der großen Halbachse und "r" die Länge der kleinen Halbachse der Ellipse.

Bei einem extrem steifen Material, also einem Material mit einem hohen Elastizitätsmodul, sollte das Verhältnis R/r gegen 1 gehen, da sich sonst die Schraube nicht in das Medium eindrehen lässt.

Bei einem extrem elastischen Material, also einem Material mit einem niedrigen Elastizitätsmodul, das eine elastische Deformation bis zu 100% zulässt, kann das Verhältnis R/r bis auf 2 ansteigen ohne das Medium plastisch zu verformen bzw. zu zerstören. Daher liegt das Verhältnis R/r vorzugsweise zwischen 1,01 und 2,0.

Geht man bei einem menschlichen Knochen von einer elastischen Deformation in einer Größenordnung von 1% aus, so kann das Verhältnis R/r bis auf 1,01 gewählt werden ohne das Knochenmaterial zu schädigen.

Eine derart im Querschnitt elliptisch dimensionierte Schraube weist im Vergleich zu den sonst im Querschnitt rund dimensionierten Schrauben einen deutlich höheren, intrinsischen Schutz gegen Lösen auf, der sich im Wesentlichen aus dem Zusammenwirken der Elastizität des Mediums oder Werkstoffs, in das die erfindungsgemäße Schraube geschraubt wird, und der Elliptizität, also dem Verhältnis der beiden Halbachsen R/r, ergibt.

Bei einem biologisch vitalen Material wie Knochen als Medium kommt noch ein weiterer Aspekt hinzu:
Geschädigtes Knochenmaterial kann sich unter bestimmten, biologischmechanischen Bedingungen regenerieren und/oder nachwachsen, d.h. das Verhältnis von R/r kann auch größer gewählt werden als es die Elastizität des Knochens grundsätzlich erlauben würde.

Es kann beispielsweise eine Ausnehmung vorgebohrt werden, die sich bzgl. des Radius an der größeren Halbachse R orientiert. Eine gewisse Primärstabilität ergibt sich daraus, dass das Gewinde im Bereich der Hauptachse eine mechanische Verbindung mit dem Werkstoff, insbesondere hier dem Knochen eingeht. Durch das Knochenwachstum bzw. die Osseointegration der Schraube im weiteren Zeitverlauf wird die Primärstabilität wesentlich verbessert, so dass eine deutlich höhere Sekundärstabilität erreicht wird. Durch das Einwachsen der Schraube in den Knochen wird auch die erfindungsgemäße Selbstsicherung der Schraube erreicht. Die Schraube kann sich aufgrund ihres elliptischen Schraubenkörpers nicht lockern oder von selbst herausschrauben.

In einer zweiten Variante und unter Ausnutzung der Elastizität des Mediums, in das die Schraube geschraubt wird, bzw. der Regenerationsfähigkeit eines vitalen Mediums, kann nicht nur der Querschnitt des Schraubenkörpers elliptisch ausgeführt sein, sondern auch bzw. nur das Längsprofil des Schraubenkörpers. Der Schraubenkörper kann also einen elliptischen Längsschnitt aufweisen.

Bezüglich der Dimensionierung dieser zweiten Variante gelten prinzipiell ähnliche Überlegungen wie bei der der ersten Variante: Der Schraubenkörper kann umso stärker von einem zylindrischen Schraubenkörper abweichen, je elastischer das Material ist, in das er eingeschraubt werden soll.

Zur Definition eines solchen Schraubenkörpers kann das Verhältnis des größten Durchmessers oder des Durchmessers an der breitesten Stelle des Schraubenkörpers D zum kleinsten Durchmesser oder zum Durchmesser an der schmalsten Stelle des Schraubenkörpers d bestimmt werden, also D/d. Dieses Verhältnis ist umso größer, je stärker der Längsschnitt des Schraubenkörpers von der üblichen zylindrischen Form abweicht. Da die zylindrische Form ein Verhältnis 1/1 = 1 aufweist, muss das Verhältnis bei Vorliegen eines elliptischen Längsschnitts größer als 1 sein.

Für die Feststellung des Durchmessers der schmalsten Stelle des Schraubenkörpers wird nur der Schraubenkörper als solches betrachtet, also der Teil, der üblicherweise ein Gewinde aufweist. Der unterste Teil einer Schraube, der häufig schmal zuläuft, um das Einschrauben zu erleichtern, wird hierbei nicht berücksichtigt. Es sei jedoch noch darauf hingewiesen, dass der zu berücksichtigende Teil des Schraubenkörpers tatsächlich kein durchgängiges Gewinde aufweisen muss.

Beide Varianten sind voneinander unabhängig, können aber auch miteinander oder mit allen gängigen Varianten des Standes der Technik kombiniert werden. Beispielsweise ist ein elliptischer Querschnitt mit einem zylindrischen Längsprofil denkbar oder ein runder Querschnitt mit einem elliptischen Längsprofil.

Die erfindungsgemäße Schraube ist insbesondere für Medien oder Werkstoffe geeignet, die eine gewisse Elastizität aufweisen und/oder vital sind, beispielsweise biologisch aktiv sind, d.h. die leben, nachwachsen, sich erneuern oder verheilen können. So eignen sich die Schrauben für die Verwendung in Menschen, Tieren und Pflanzen, insbesondere Bäume, Sträucher oder auch Bambus.

Die Schrauben werden als Knochenschrauben verwendet, beispielsweise um Implantate zu befestigen. Ohne Beschränkung der Allgemeinheit eignen sich die Schrauben zur Befestigung von Wirbelsäulenimplantaten zur Fusionierung von Wirbelkörpern oder zum Ersatz der Bandscheiben, zur Befestigung von Traumaprodukten jeglicher Art oder auch Dentalimplantaten.

Bezüglich der genannten elastischen Werkstoffe eignen sich beispielsweise besonders polymerbasierte Materialien.

Die genannten Beispiele sind nicht beschränkend gemeint, sondern dienen lediglich der Erläuterung des Grundprinzips der Erfindung.

Die Erfindung ist nicht beschränkt auf spezielle Gewindeformen. Alle gängigen Formen aus dem Stand der Technik können eingesetzt werden, die jedoch den Erfordernissen des gewählten Schraubenwerkstoffs Rechnung tragen sollen. Insbesondere eignen sich auch alle Gewindeformen, die bei Spongiosaschrauben oder Kortikalisschrauben zur Anwendung gelangen.

Gleiches gilt für Schraubenkopf und Schraubenantrieb, wobei sich erfindungsgemäß besonders elliptisch geformte Schraubenköpfe eignen, die bei Versenkung in das Medium zusätzlich zur erfindungsgemäßen Selbstsicherung beitragen. Eine weitere bevorzugte Schraubenkopf-Ausführungsform hat an ihrer Unterseite, also der Seite, die mit dem Werkstoff mechanisch in Verbindung steht, Zähne oder Widerhaken, die ein Hineindrehen problemlos ermöglichen, aber dem Herausdrehen der Schraube Widerstand entgegensetzen, in dem sie sich mit dem Material verhaken.

Als Schraubenmaterial eignen sich alle bekannten Werkstoffe, die nach dem Stand der Technik eingesetzt werden, insbesondere aus der Klasse der metallischen Werkstoffe, der Kunststoffe, aber auch der keramischen und Keramik-basierten Werkstoffe.

Zur letztgenannten Werkstoffgruppe zählen insbesondere alle Aluminiumoxid- und Zirkonoxid-basierten Werkstoffe sowie auf diesen Materialien basierende Verbundwerkstoffe, aber auch nichtoxidische keramische Werkstoffe, wie beispielsweise Siliziumnitrid- oder Siliziumcarbid-basierte Werkstoffe sowie auf diesen Materialien basierende Verbundwerkstoffe.

Vorteilhaft im Sinne der Erfindung ist es, die Oberfläche des Schraubenkörpers durch geeignete Techniken zu erhöhen und/oder diese Oberfläche durch geeignete Beschichtungen so zu modifizieren, dass eine möglichst hohe Osseointegrationsrate eingestellt wird. Beispielsweise kann zumindest ein Teil einer Oberfläche des Schraubenkörpers aufgeraut sein.

In dieser Hinsicht eignen sich zur Oberflächenbehandlung prinzipiell alle aus dem Stand der Technik bekannten Verfahren, die mechanische und/oder chemisch basierte Methoden beinhalten können. Mechanische Verfahren zur Veränderung der Rauheit der Oberfläche umfassen beispielsweise Verfahren wie Schleifen, Polieren, Strahlen, chemisches Ätzen und Plasmabeschichtung und/oder Plasmaaktivierung.

Speziell für keramische Schraubenwerkstoffe eignen sich Aufsprühverfahren von zusätzlichen keramischen Lagen zur Erzeugung einer größeren Oberfläche. Beispielsweise kann ein keramischer Schlicker aufgebracht werden, der entweder schon Porosierungsmittel enthält, oder aber die Porosierungsmittel werden nach dem Aufbringen des Schlickers in dessen Oberfläche eingebracht und anschließend, vorzugsweise rückstandsfrei, ausgebrannt. Gleiches funktioniert natürlich auch mit allen anderen keramischen Verfahren, die eine offen-poröse keramische Matrix erzeugen.

Zur Funktionalisierung der Oberflächen zumindest des Schraubenkörpers im Sinne einer Erhöhung der Osseointegrationfähigkeit bzw. der Osseointegrationsrate eignen sich für die erfindungsgemäßen Schrauben alle Arten von die Osseointegration fördernden Beschichtungen, beispielsweise basierend auf Hydroxylapatit oder Tricalciumphosphat oder ähnlichen biokeramischen Zusammensetzungen, alle Arten von Bioglasbeschichtungen und alle Arten von Molekülen, die ein effektives Andocken von Knochenzellen an die Schraubenoberfläche sicher und dauerhaft garantieren. Dem Fachmann sind entsprechende Substanzen aus dem Stand der Technik bekannt.

Auch metallische Beschichtungen wie dünne Titan-Plasmabeschichtungen oder auf andere Art physikalisch abgeschiedene Titanbeschichtungen können den erfindungsgemäßen Gedanken unterstützen.

Die Erfindung wird im Folgenden anhand der Zeichnungen näher erläutert. Die Zeichnungen zeigen:
- Fig. 1:: Erfindungsgemäße Schraube im Längsschnitt
- Fig. 2:: Schnitt durch einen Schraubenkörper mit einem elliptischen Querschnitt
- Fig. 3:: Schnitt durch einen Schraubenkörper mit einem elliptischen Längsschnitt

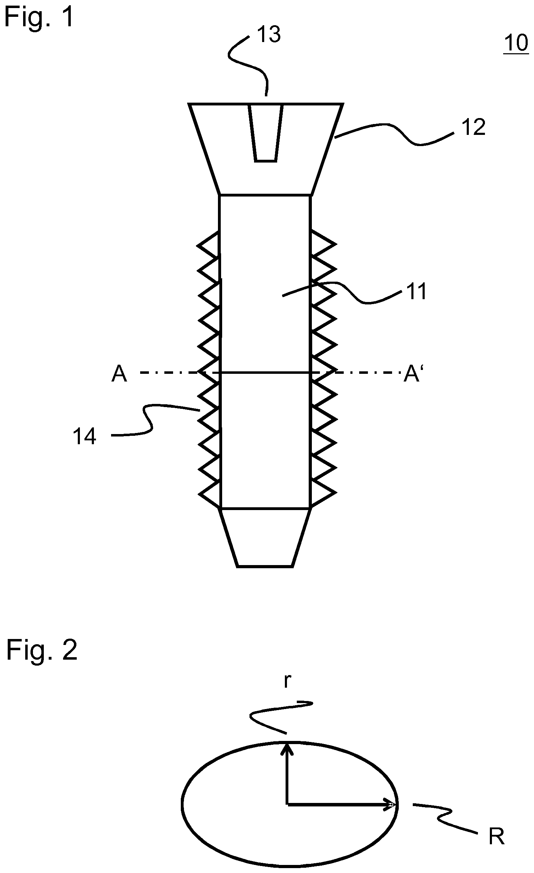

Figur 1 zeigt eine erfindungsgemäße Schraube 10 im Längsschnitt. Die Schraube 10 weist einen Schraubenkörper 11 mit einem Gewinde 14 und einen Schraubenkopf 12 mit einem Kopfantrieb 13 auf. Der Schraubenkörper 11 ist im Längsschnitt zylindrisch.

Der Schraubenkörper 11 ist im Querschnitt elliptisch geformt. Ein entsprechender Querschnitt ist zur Verdeutlichung in Fig. 2 dargestellt, wobei die Linie A-A' die Schnittebene in Fig. 1 zeigt.

Der elliptische Querschnitt des Schraubenkörpers 11 weist eine Haupt- und eine Nebenachse auf, wobei die Längen der jeweiligen Halbachsen R und r die Form des elliptischen Querschnitts definieren. Das Verhältnis zwischen R und r, R/r, liegt bevorzugt zwischen 1,01 und 2,0.

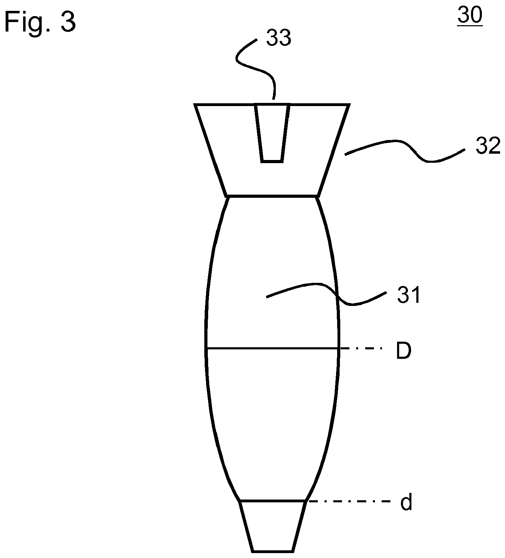

Figur 3 zeigt eine weitere Schraube 30. Diese weist wie schon im Beispiel nach Fig. 1 einen Schraubenkörper 31 und einen Schraubenkopf 32 mit einem Kopfantrieb 33 auf. Die Schraube 30 weist natürlich auch zumindest auf Teilen des Schraubenkörpers 31 ein Gewinde auf, das jedoch in der Zeichnung nicht dargestellt ist.

Im Gegensatz zu dem in Fig. 1 gezeigten Beispiel hat die Schraube 30 keinen im Längsschnitt zylindrischen Schraubenkörper, sondern einen im Längsschnitt elliptischen Schraubenkörper 31.

Der Querschnitt des Schraubenkörpers kann, wie bei Schrauben normalerweise üblich, rund sein. Gemäß der Erfindung ist der Schraubenkörper aber im Querschnitt auch elliptisch. In diesem Fall sind also sowohl der Quer- als auch der Längsschnitt durch den Schraubenkörper elliptisch.

Vorzugsweise ist das Verhältnis von D/d, das aus dem Verhältnis der Durchmesser an der breitesten und der schmalsten Stelle oder auch aus dem größten und dem kleinsten Durchmesser des Schraubenkörpers gebildet wird, ist größer als 1.

## Patentansprüche

1. Knochenschraube (10, 30), umfassend einen Schraubenkörper (11, 31) mit einem Gewinde (14), wobei der Schraubenkörper (11, 31) elliptisch ist, **dadurch gekennzeichnet, dass** der Schraubenkörper (11, 31) einen elliptischen Querschnitt und einen elliptischen Längsschnitt aufweist und zumindest ein Teil des Schraubenkörpers (11, 31) mit einer Beschichtung, insbesondere mit einer die Osseointegration fördernden Beschichtung, versehen ist.

2. Knochenschraube (10, 30) nach Anspruch 1, **dadurch gekennzeichnet, dass** ein Verhältnis einer Länge R einer großen Halbachse zu einer Länge r einer kleinen Halbachse des elliptischen Querschnitts, also R/r, zwischen 1,01 und 2,0 liegt.

3. Knochenschraube (10, 30) nach 1, **dadurch gekennzeichnet, dass** das Verhältnis des größten Durchmessers des Längsschnitts D zum kleinsten Durchmesser des Längsschnitts d größer als 1 ist.

4. Knochenschraube (10, 30) nach einem der vorstehenden Ansprüche, **dadurch gekennzeichnet, dass** zumindest der Schraubenkörper (11, 31) aus einem metallischen oder einem keramischen Werkstoff, einem keramischen Verbundwerkstoff oder einem Kunststoff besteht.

5. Knochenschraube (10, 30) nach einem der vorstehenden Ansprüche, **dadurch gekennzeichnet, dass** zumindest ein Teil einer Oberfläche des Schraubenkörpers (11, 31) aufgeraut ist.

## Claims

1. Bone screw (10, 30), comprising a screw body (11, 31) having a thread (14), the screw body (11, 31) being elliptical, **characterized in that** the screw body (11, 31) has an elliptical cross section and an elliptical longitudinal section and at least one part of the screw body (11, 31) is provided with a coating, in particular with a coating promoting osseointegration.

2. Bone screw (10, 30) according to claim 1, **characterized in that** a ratio of a length R of a semi-major axis to a length r of a semi-minor axis of the elliptical cross section, i.e. R/r, is between 1.01 and 2.0.

3. Bone screw (10, 30) according to 1, **characterized in that** the ratio of the largest diameter of the longitudinal section D to the smallest diameter of the longitudinal section d is greater than 1.

4. Bone screw (10, 30) according to any of the preceding claims, **characterized in that** at least the screw body (11, 31) consists of a metal or a ceramic material, a ceramic composite material or a plastics material.

5. Bone screw (10, 30) according to any of the preceding claims, **characterized in that** at least one part of a surface of the screw body (11, 31) is roughened.

## Revendications

1. Vis à os (10, 30) comprenant un corps de vis (11, 31) avec un filetage (14), le corps de vis (11, 31) étant elliptique, **caractérisée en ce que** le corps de vis (11, 31) présente une section transversale elliptique et une section longitudinale elliptique et au moins une partie du corps de vis (11, 31) munie d'un revêtement, en particulier un revêtement favorisant une ostéointégration.

2. Vis à os (10, 30) selon la revendication 1, **caractérisée en ce qu'**un rapport d'une longueur R d'un demi-grand axe à une longueur r d'un demi-petit axe de la section transversale elliptique, c'est-à-dire R/r, est compris entre 1,01 et 2,0.

3. Vis à os (10, 30) selon la revendication 1, **caractérisée en ce que** le rapport du plus grand diamètre de la section longitudinale D au plus petit diamètre de la section longitudinale d est supérieur à 1.

4. Vis à os (10, 30) selon l'une des revendications précédentes, **caractérisée en ce qu'**au moins le corps de vis (11, 31) est constitué d'un matériau métallique ou céramique, d'un matériau composite céramique ou d'une matière plastique.

5. Vis à os (10, 30) selon l'une des revendications précédentes, **caractérisée en ce qu'**au moins une partie d'une surface du corps de vis (11, 31) est rendue rugueuse.
